# EUROPÄISCHE PATENTSCHRIFT

(11) **EP 2 228 098 B1**
(45) Veröffentlichungstag und Bekanntmachung des Hinweises auf die Patenterteilung: **04.05.2011**
(21) Anmeldenummer: 09003603.9
(22) Anmeldetag: 12.03.2009
(51) Int. Cl.: A61N 5/06

(54) **Liegescheibe für ein Bräunungsgerät, Bräunungsgerät und Verfahren zur Herstellung einer Liegescheibe**
Lying disc for a tanning device, tanning device and method for producing a lying disc
Plaque pour s'allonger destinée à un appareil de bronzage, appareil de bronzage et procédé de fabrication d'une plaque pour s'allonger

(43) Veröffentlichungstag der Anmeldung: 15.09.2010
(73) Patentinhaber: SP-GmbH & Co. KG, 50259 Pulheim (DE)
(72) Erfinder: Müller, Wolfgang, 50968 Köln-Marienburg (DE)
(74) Vertreter: Weidener, Jörg Michael

(56) Entgegenhaltungen:
- WO-A1-97/00709
- DE-A1- 3 717 393
- DE-A1-102004 051 939
- DE-A1-102006 010 583
- US-A- 4 381 136

## Beschreibung

Die Erfindung betrifft eine Liegescheibe für ein Bräunungsgerät, mit einem UV-durchlässigen Scheibenkörper aus Kunststoff zur Auflage eines Nutzers. Desweiteren betrifft die vorliegende Erfindung ein Bräunungsgerät mit einer Liegescheibe der vorgenannten Art sowie ein Verfahren zur Herstellung einer Liegescheibe für ein Bräunungsgerät.

Bräunungsgeräte der vorgenannten Art und dabei verwendete Liegescheiben sind aus dem Stand der Technik bereits seit langem bekannt. Bei diesen Scheiben kann es sich um verformte oder auch um ebene Scheiben handeln, auf die sich ein Nutzer zur Bräunung legt. Ein wesentliches Kriterium bei Liegescheiben der vorgenannten Art ist, daß der Scheibenkörper UV-durchlässig ist. Mit der UV-Durchlässigkeit ist grundsätzlich eine Durchlässigkeit im UV/A-(400 bis 320 nm) und UV/B-Bereich (320 bis 280 nm) gemeint. Als Material der Liegescheiben wird üblicherweise Kunststoff verwendet, da das Liegen auf einem Kunststoff von einem Nutzer angenehmer empfunden wird als das Liegen auf Glas.

Bei Bräunungsgeräten der in Rede stehenden Art ist in der Praxis festgestellt worden, daß durch die thermische, chemische und mechanische Belastung nicht nur das optische Erscheinungsbild der Liegescheiben beeinträchtigt wird, sondern sich nach einer gewissen Betriebszeit auch eine Funktionsbeeinträchtigung durch eine verschlechterte UV-Durchlässigkeit ergibt, so daß das Bräunungsergebnis nicht hinreichend gewährleistet werden kann.

Bei Untersuchungen, die von der Anmelderin durchgeführt worden sind, ist festgestellt worden, daß die Liegescheiben häufig durch die verwendeten chemischen Reiniger, die üblicherweise nach einer Nutzung eines Bräunungsgeräts zur Reinigung der Liegescheibe eingesetzt werden, und auch durch den menschlichen Schweiß in Verbindung mit der mechanischen Belastung durch die Bewegung des Nutzers auf der Liegescheibe dazu führt, daß die Oberfläche der Liegescheibe nicht nur matt wird, sondern sich auch Mikrorisse ergeben. Es ist festgestellt worden, daß sich nach dem ersten Auftreten von Mikrorissen dieses Problem immer mehr verstärkt. Der Grund hierfür besteht darin, daß der Schweiß und der verwendete chemische Reiniger in die Mikrorisse eindringt und eine Rißfortsetzung und fortlaufendes Entstehen weiterer Mikrorisse begünstigt. Auch die zunehmende Versprödung der Liegescheibe aufgrund der UV-Bestrahlung begünstigt die Rißbildung. Als Folge dieser Problematik ist es so, daß üblicherweise nach etwa 2000 Betriebstunden die Liegescheibe ausgetauscht werden muß. Da die Liegescheibe letztlich eines der teuersten, wenn nicht das teuerste Austauschteil an einem Bräunungsgerät darstellt, bedeutet dieser Austausch für den Betreiber des Bräunungsgerätes einen erheblichen Kostenanfall,

Aus der DE 10 2006 010 583 A1 ist eine Besonnungseinrichtung bekannt, bei der eine Liegescheibe für den Nutzer vorgesehen ist. Zwischen der Liegescheibe und den UV-Strahlem befindet sich ein Folienfilter, der unterseitig an der Liegescheibe an seinen Rändern mit Klebeband fixiert ist. Um den Folienfilter nach einer vorgesehenen Betriebszeit auswechseln zu können, sollte dieser in einfacher Weise austauschbar sein.

Aus der DE 10 2004 051 939 A1 ist bereits ein beschichteter Kunststoff-Formkörper und ein Verfahren zu dessen Herstellung bekannt. Dieser bekannte Kunststoff-Formkörper kann unter anderem als Liegefläche einer Sonnenbank Anwendung finden. Die Beschichtung dient bei dem bekannten Kunststoff-Formkörper dazu, ein verbessertes Eigenschaftsprofil zur Verfugung zu stellen. Dementsprechend soll die Beschichtung möglichst kratzfest, transparent, witterungsbeständig und langlebig sein und dabei eine möglichst hohe Reißdehnung und eine möglichst hohe Haftung auf dem zu beschichtenden Substrat aufweisen, damit ein Ablösen der Beschichtung unter mechanischer Beanspruchung bestmöglich vermieden wird.

Aufgabe der vorliegenden Erfindung ist es, eine Liegescheibe für ein Bräunungsgerät zur Verfügung zu stellen, die über eine möglichst lange Betriebsdauer einen hinreichenden Verschleißschutz und eine ausreichende Transmission gewährleistet.

Zur Lösung des vorgenannten Problems sind bezüglich der Liegescheibe die im Patentanspruch 1 angegebenen Merkmale vorgesehen.

Durch die UV-durchlässige Beschichtung auf der Auflage- oder Liegeseite der Liegescheibe ergibt sich zunächst einmal ein wirksamer Verschleißschutz. Die Beschichtung verhindert je nach Beschichtungsdicke und Beschichtungsart jedenfalls für einen vorgegebenen Zeitraum, daß Reinigungsmittel und/oder menschlicher Schweiß an die Oberseite des Scheibenkörpers gelangen und zu Beeinträchtigungen führen können. Des weiteren ist auf der Unterseite der Liegescheibe, die der Auflage- oder Liegeseite abgewandt ist, ebenfalls eine UV-durchlässige Beschichtung vorzusehen. Eine Beschichtung an dieser Stelle führt nach Untersuchung der Anmelderin dazu, daß die Liegescheibe durch die UV-Bestrahlung weniger schnell versprödet, so daß die Rißbildung erheblich verzögert wird. In jedem Fall kann die mögliche Nutzungszeit der Liegescheibe durch die vorgenannte beidseitige Beschichtung nicht unerheblich erhöht werden. Außerdem wirkt sich die erhöhte Nutzungsdauer kostenmäßig günstig aus, da die durch die Beschichtung entstehenden Kosten geringer sind als der Nutzen, der aus der längeren Nutzungsdauer resultiert. Im übrigen erfährt das mit einer erfindungsgemäßen Liegescheibe ausgestattete Bräunungsgerät keine Funktionsbeeinträchtigung, da die Beschichtung, wie der Scheibenkörper auch, UV-durchlässig ist. Da die Aufbringung einer Beschichtung einen zusätzlichen Verfahrensschritt bei der Herstellung darstellt, der mit Kosten verbunden ist, und es grundsätzlich auch möglich wäre, der vorstehend genannten Problematik in anderer Weise zu begegnen, nämlich beispielsweise durch Auswahl eines anderen, härteren oder verschleißfesteren kunststoffmaterial als bisher, Verwendung von Glasliegescheiben mit entsprechender Beheizung oder ähnlichem, erscheint die erfindungsgemäße Lösung nicht ohne weiteres naheliegend.

Überraschenderweise ist bei Versuchen im Zusammenhang mit der Erfindung festgestellt worden, daß sich gegenüber einer einseitigen Beschichtung auch in Langzeitversuchen verbesserte Transmissionswerte ergeben, wenn der Scheibenkörper beidseitig, also ober- und unterseitig beschichtet ist. Dieses Ergebnis war an sich nicht zu erwarten, da man zunächst angenommen hatte, daß eine beidseitige Beschichtung grundsätzlich tendenziell eher zu einer Verschlechterung der Transmissionswerte führen würde.

Um den Anforderungen zur Bräunung entsprechen zu können, ist die Beschichtung derart ausgebildet bzw. ausgewählt, daß sie bei einer Strahlung im Wellenlängenbereich zwischen 280 nm und 450 nm einen Transmissionsgrad von wenigstens 50% aufweist. Vorzugsweise sollte der Transmissionsgrad in dem vorgenannten Wellenlängenbereich zwischen 60 und 90% liegen. Da UV-Strahlung gerade ab einer Wellenlänge von 295 nm in erheblichem Maße zur Bräunung beiträgt, ist es besonders bevorzugt, wenn ab dieser Wellenlänge ein Transmissionsgrad von wenigstens 70% gegeben ist. Mit dem Transmissionsgrad bezeichnet man dabei diejenige Strahlungsmenge, die von der aufgegebenen UV-Strahlung durch die Bräunungsleuchten durch die UV-Beschichtung nach Hindurchtreten durch den Scheibenkörper hindurchgelangt, Ein 70%iger Transmissionsgrad bedeutet im Ergebnis damit, daß 30% der unterseitig auf die Liegescheibe aufgebrachten Strahlungsleistung nicht durch die Liegescheibe einschließlich Beschichtung hindurchgelangt.

Im übrigen ist festgestellt worden, daß sich - verglichen mit einer Liegescheibe ohne Beschichtung und unter Berücksichtigung der sich durch die Beschichtung ergebenden Kosten - die Erfindung bereits dann positiv auswirkt, wenn die Beschichtung derart ausgebildet ist, daß der vorgenannte Transmissionsgrad über eine Bestrahlungsdauer von wenigstens 100 Stunden konstant bleibt. Besonders bevorzugt ist es, wenn die Beschichtung derart ausgebildet ist, daß der vorgenannte Transmissionsgrad über einen Zeitraum von bis zu 1000 Stunden nicht unterschritten wird. Das vorgenannte Merkmal bedeutet letztlich, daß zwar über die Liegedauer ein höherer Transmissionsgrad möglich ist, der Transmissionsgrad jedoch die vorgenannte untere Grenze innerhalb des genannten Zeitraums nicht unterschreitet.

Grundsätzlich eignen sich unterschiedlichste UV-durchlässige Beschichtungsmaterialien zur Verwendung bei einer erfindungsgemäßen Liegescheibe. Bei Versuchen ist festgestellt worden, daß besonders günstige Eigenschaften dann erzielt werden, wenn die Beschichtung ein kunststoffbasiertes Lacksystem aufweiset, und zwar insbesondere auf Basis zumindest eines organischen und/oder anorganischen Polymers. Ganz besonders gute Ergebnisse sind in Verbindung mit einem Lacksystem ermittelt worden, das Silikon- und/oder Polysiloxan enthält oder hieraus besteht. Mit Silikon- und/oder Polysiloxan basierten Lacksystemen sind im Ergebnis in Langzeitversuchen hohe Transmissionsgrade auch nach entsprechender Belastung der Auflageseite der Liegescheibe erzielt worden.

Besonders günstig ist insbesondere die Verwendung einer Materialkombination von PMMA als Scheibenkörpermaterial mit einem polysiloxanbasierten Lacksystem, wobei es sich versteht, daß der Scheibenkörper grundsätzlich auch aus jedem anderen thermoplastischen Kunststoff bestehen kann und das Lacksystem, wie zuvor bereits ausgerührt worden ist, nicht notwendigerweise polysiloxanbasiert sein muß.

Im übrigen sollte zur Erzielung eines hinreichenden Transmissionsgrades bzw. zur Vermeidung von Transmissionsverlusten die Dicke des Scheibenkörpers zwischen 6 und 12 mm und insbesondere bei etwa 8 mm liegen.

Des weiteren ist es von besonderem Vorteil, daß das Lacksystem mit einem Haftvermittler kombiniert ist. Der Haftvermittler sorgt für eine verbesserte Haftung des Beschichtungsmaterials an der Auflageseite des Scheibenkörpers. Zwar ist es grundsätzlich auch möglich, den Haftvermittler vor dem eigentlichen Beschichtungsvorgang auf die zu beschichtende Seite der Liegescheibe aufzubringen, jedoch ist festgestellt worden, daß die gleichzeitige Applikation des Lacksystems mit dem darin vorgesehenen Haftvermittler letztlich zu einem besseren Haftergebnis und auch zu einer beschichteten Liegescheibe mit hohen Transmissionswerten führt.

Insbesondere bei einem aus PMMA bestehenden bzw. PPMA aufweisenden Scheibenkörper hat es sich gezeigt, daß der Haftvermittler aus einem Material bestehen bzw. ein solches aufweisen sollte, daß zu einer "Anrauhung" der zu beschichtenden Oberfläche im nm- und/oder um-Bereich führt Gerade bei einem siloxanbasierten Lacksystem als Beschichtungsmaterial ergibt sich dann ein sehr gutes Beschichtungsergebnis.

Zur Erzielung des vorgenannten Beschichtungserfolges ist es günstig, wenn der Haftvermittler ausgewählt ist aus der Gruppe von Säuren, insbesondere organischen Säuren und vorzugsweise Essigsäure, Aldehyden, Ketonen, Alkoholen, Estern, Äthern, Lösungsmitteln sowie deren Mischungen und Kombinationen.

Wie zuvor bereits ausgeführt worden ist, ist es bei der erfindungsgemäßen Liegescheibe einerseits wichtig, daß sich eine möglichst hohe Verschleißbeständigkeit ergibt. Zum anderen ist wichtig, daß sich eine hohe UV-Durchlässigkeit bzw. ein hoher Transmissionsgrad ergibt. Die beiden vorgenannten Ziele sind an sich gegenläufig, da eine sehr dicke Beschichtung zwar verschleißfester ist und zu einer längeren Betriebsdauer der Liegescheibe führt. Jedoch ist eine sehr dicke Beschichtung in der Regel mit einem relativ geringen Transmissionsgrad verbunden. Um beiden Anforderungen hinreichend gerecht zu werden, ist festgestellt, daß die Schichtdicke der Beschichtung kleiner 50 µm sein sollte. Bevorzugt liegt die Schichtdicke zwischen 2 und 10 µm.

Im übrigen betrifft die vorliegende Erfindung auch ein Bräunungsgerät mit einer Liegescheibe der vorgenannten Art, das heißt mit allen zuvor angegebenen und auch noch nachfolgend beschriebenen Merkmalen. Hinzuweisen ist darauf, daß das Bräunungsgerät jede Form aufweisen kann, es kann sich beispielsweise um ein sogenanntes Tunnelgerät handeln. Entscheidend ist lediglich, daß es eine Liegescheibe zur Auflage eines Nutzers zum Bräunen aufweist.

Darüber hinaus betrifft die vorliegende Erfindung ein Verfahren zur Herstellung einer Liegescheibe für ein Bräunungsgerät, wobei die Liegescheibe mit einem UV-durchlässigen Scheibenkörper aus Kunststoff zur Auflage eines Nutzers versehen ist und wobei der Scheibenkörper auf seiner Oberseite und seiner Unterseite mit einer UV-durchlässigen Beschichtung beschichtet wird. Auf die erfindungsgemäßen Vorteile ist bereits eingangs eingegangen worden, so daß an dieser Stelle darauf Bezug genommen werden kann.

Zuvor ist bereits ausgerührt worden, daß die Beschichtung sowohl ein- als auch beidseitig vorgesehen sein kann. Eine ein- oder beidseitige Beschichtung kann grundsätzlich im Flutverfahren vorgenommen werden. Beim Flutverfahren wird die Liegescheibe im stehenden oder schrägen Zustand angeordnet und anschließend läuft das flüssige Beschichtungsmaterial von oben nach unten an der Auflagefläche des Scheibenkörpers entlang.

Das Flutverfahren eignet sich auch deshalb gut zur Beschichtung, da etwaig noch an der Liegescheibe anhaftende Staubpartikel und Verschmutzungen von dem beim Fluten ablaufenden Beschichtungsmaterial erfaßt werden und im günstigsten Falle ablaufen.

Statt des Flutverfahrens kann, insbesondere wenn die Liegescheibe beidseitig beschichtet werden soll, auch eine Beschichtung im Sprüh- oder Tauchverfahren vorgenommen werden. Beim Tauchverfahren wird die Scheibe dann in ein entsprechendes Bad mit flüssigem Beschichtungsmaterial eingetaucht. Beim Sprühverfahren wird die Scheibe ein- oder beidseitig gleichzeitig oder nacheinander besprüht.

Die Dauer des Beschichtungsvorganges hängt zum einen von der Größe der Liegescheibe und zum anderen von der Viskosität des Beschichtungsmaterials ab. Bei Versuchen, die durchgeführt worden sind, sind Beschichtungszeiten jedenfalls beim Flutverfahren pro Liegescheibe und Seite von 15 Minuten nicht überschritten worden. Üblicherweise liegt die Beschichtungsdauer einer Seite einer durchschnittlich großen Liegescheibe bei etwa 5 bis 10 Minuten.

Zur Gewährleistung eines automatischen Herstellungsverfahrens bietet es sich an, die Scheibenkörper der Liegescheiben vor ihrer Beschichtung in eine Transporteinrichtung zu hängen. Hierzu können an der Liegescheibe vorhandene Bohrungen genutzt oder aber auch, bei noch nicht endbearbeiteten Scheiben, Bohrungen insbesondere im Randbereich der Liegescheibe gesetzt werden. Das Einhängen der Liegescheiben in die Transporteinrichtung sorgt jedenfalls dafür, daß sich eine definierte Anordnung der Liegescheibe bei der Herstellung ergibt, was für die Automatisierung des Prozesses wichtig ist.

Vor der eigentlichen Beschichtung bietet es sich an, die zu beschichtende Oberfläche der Liegescheibe zu reinigen, um anhaftende Partikel zu beseitigen. In diesem Zusammenhang ist es günstig, daß die Auflageseite mit einem Reinigungsmittel, insbesondere einem Alkohol und vorzugsweise Ethanol, gereinigt wird. Dies kann durch manuelles oder automatisches Abwischen der Oberfläche erfolgen.

Ergänzend oder alternativ zu der vorgenannten Reinigung oder in Verbindung damit kann es sich anbieten, die Auflageseite des Scheibenkörpers ergänzend mit ionisierter Druckluft zu reinigen. Die ionisierte Luft kann mit einem Druck von ca. 8 bar aufgebracht werden. Hierdurch kann nicht nur anhaftender Feinstaub entfernt werden, die zu beschichtende Oberfläche wird auch statisch entladen, was das Anhaften des Beschichtungsmaterials begünstigt.

Nach dem Beschichtungsvorgang insbesondere mit dem vorgenannten Lacksystem und dem speziellen Haftvermittler bietet es sich an, das Lacksystem bei Raumtemperatur ablüften zu lassen, bis es trocken ist. Dies kann ohne eine ergänzende Beheizung und insbesondere bei Raumtemperatur erfolgen. Es ist festgestellt worden, daß sich bei einem derartigen Ablüften bzw. Trocknen eine ausgesprochen gute Anhaftung der Beschichtung an der Oberseite des Scheibenkörpers ergibt.

Verfahrenstechnisch bietet es sich im übrigen an, daß nach dem Beschichtungsvorgang das gesamte System, das heißt der Scheibenkörper und auch die Beschichtung, thermisch aushärtet. Es ist festgestellt worden, daß das thermische Aushärten bei einer Temperatur größer 60°, und insbesondere zwischen 80° bis 100° erfolgen sollte.

Nachfolgend werden Ausführungsbeispiele der Erfindung anhand der Zeichnung erläutert. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt
- Fig. 1: eine perspektivische schematische Ansicht eines erfindungsgemä- ßen Bräunungsgeräts,
- Fig. 2: eine Draufsicht auf eine Ausführungsform einer erfindungsgemäßen Liegescheibe,
- Fig. 3: eine schematische Darstellung des Schichtaufbaus einer erfin- dungsgemäßen Liegescheibe und
- Fig. 4: eine schematische Darstellung des erfindungsgemäßen Beschich- tungsverfahrens.

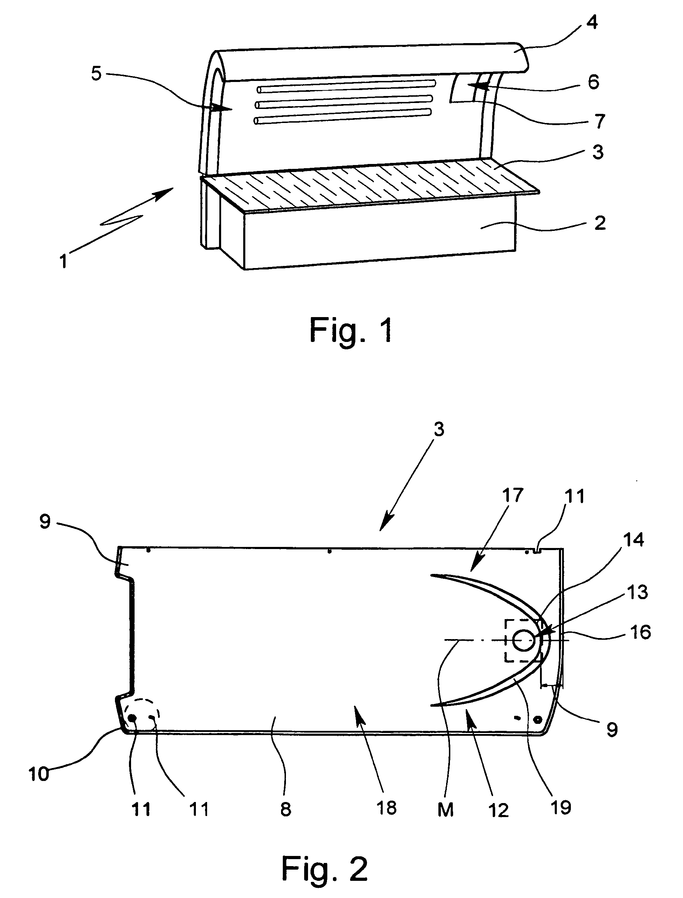

In Fig. 1 ist ein als Sonnenbank ausgebildetes Bräunungsgerät 1 zur UV-Bestrahlung der menschlichen Haut dargestellt. Bei dem Bräunungsgerät 1 handelt es sich um ein sogenanntes Tunnelgerät, das einen Unterbau 2 mit einer Liegescheibe 3 und ein am Unterbau 2 angelenktes, schwenkbares Oberteil 4 aufweist. Das Oberteil 4 ist auf den Unterbau 2 herabschwenkbar, so daß sich ein Tunnel ergibt, in dem sich während des Betriebes der Nutzer befindet. Unterhalb der Liegescheibe 3 und im Oberteil 4 befinden sich vorliegend langgestreckte Leuchtstoff-Bräunungsleuchten 5. Hinter den Bräunungsleuchten 5 befinden sich jeweils Reflektoren, die im einzelnen nicht dargestellt sind.

Im Gesichtsbereich des Oberteils 4 des Bräunungsgeräts 1 befindet sich ein Gesichtsbräuner 6. Der Gesichtsbräuner 6 weist eine äußere Schutzscheibe 7 auf.

Hinter der Schutzscheibe 7 befindet sich eine Bräunungslampe mit einem Reflektor und einer Filterscheibe als Gesichtsbräuner.

Im übrigen versteht es sich, daß statt der dargestellten Ausführungsform des Bräunungsgeräts 1 auch andere Ausführungsformen von Bräunungsgeräten möglich sind, die vorliegende Erfindung also nicht auf die dargestellte Ausführungsform beschränkt ist.

Während die in Fig. 1 dargestellte Liegescheibe 3 eine ebene Form aufweist, ist die Liegescheibe 3 bei der in Fig. 2 dargestellten Ausführungsform verformt. Die Liegescheibe 3 selbst weist einen Scheibenkörper 8 mit einer rechteckigen Form auf, wobei der Scheibenkörper 8 aus einem Kunststoff, insbesondere PMMA besteht. Die Liegescheibe 3 weist vorliegend eine Gesamtlänge einschließlich der beiden am Fußende vorgesehenen Vorsprüngen 9, 10 von etwa 2,15 m bei einer Gesamtbreite von ca. 93 cm auf. Im übrigen sind insbesondere im Randbereich eine Reihe von Öffnungen 11 zur Positionierung und Befestigung der Liegescheibe 3 am Unterbau 2 des Bräunungsgeräts 1 oder auch eines anderen Bräunungsgeräts vorgesehen.

Bei der dargestellten Ausführungsform ist es nun so, daß im Kopfbereich 12 des Scheibenkörpers 8 eine muldenförmige Vertiefung 13 unterhalb des Zentrums 14 des Strahlungsfeldes des Gesichtsbräuners 6 angeordnet, und zwar im Bereich des nach unten projizierten Zentrums 14. Das Zentrum 14 ist vorliegend lediglich schematisch als Rechteck dargestellt. Es versteht sich, daß das Zentrum 14 jede beliebige Form haben kann. Im Zentrum 14 ist die Strahlungskonzentration bzw. Intensität des Strahlungsfeldes am höchsten.

Bei der in Fig. 2 dargestellten Ausführungsform weist die Vertiefung 13 eine kreisrunde Form auf. Der Durchmesser beträgt ca. 10 cm. Die Vertiefung 13 hat dabei eine Tiefe von etwa 20 mm. Letztlich hat die Vertiefung eine Kugelsegmentform, die an die Hinterkopfform des Nutzers angepaßt ist. Der lichte Abstand 15 der Vertiefung 13 zur oberen Stirnkante 16 beträgt etwa 11 cm.

Im übrigen befindet sich im Kopfbereich 12 des Scheibenkörpers 8 eine Erhöhung 17. Die Erhöhung 17 setzt am Übergang des mittigen Bereichs 18 zum Kopfbereich an und erhöht sich kontinuierlich, wobei etwa im Bereich der höchsten Stelle der Erhöhung 17 die Vertiefung 13 vorgesehen ist. Die Erhöhung 17 weist eine im wesentlichen parabelartige Form auf, wobei die Parabel zum mittigen Bereich 18 hin geöffnet und abfallend ist. Nach außen hin fällt die Erhöhung 17 stark ab. Diese Abschrägung ist mit dem Bezugszeichen 19 gekennzeichnet. Die maximale Höhe der Erhöhung 17 beträgt vorliegend etwa 6 cm, wobei die Erhöhung 17 grundsätzlich eine maximale Höhe zwischen 2 cm und 10 cm haben kann. Die Vertiefung 13 befindet sich mittig auf der Mittelachse M der Parabel, und zwar im Bereich der Parabelspitze. Da die Mittelachse M der Parabel auch die Mittelachse der Vertiefung 13 bildet, entspricht diese gleichzeitig der Mittelachse des Nutzers beim Bräunungsvorgang.

Wie sich im übrigen aus Fig. 2 ergibt, ist der Scheibenkörper 8 im Anschluß an den Kopfbereich 12, der letztlich im wesentlichen das obere Drittel des Scheibenkörpers 8 einnimmt, eben bzw. unprofiliert, so daß für den Nutzer keine sonstigen Fixierungspunkte mit Ausnahme der Vertiefung 13 auf der Liegescheibe 8 gegeben sind.

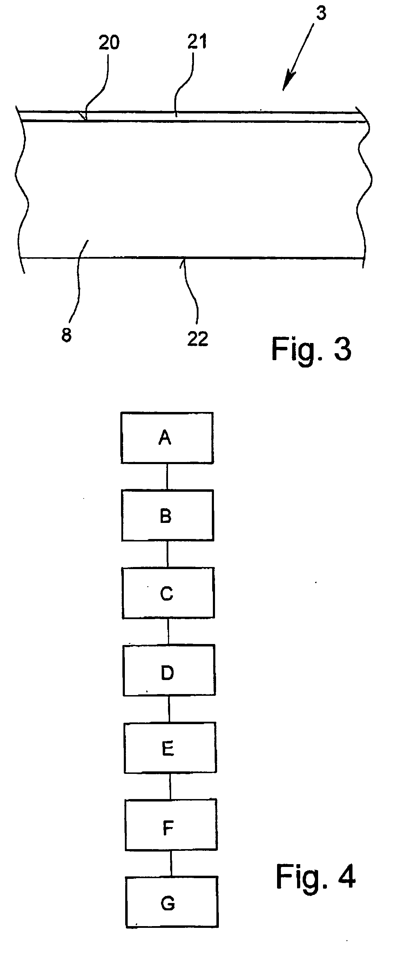

Bei der Liegescheibe 3, deren Scheibenkörper 8 UV-durchlässig ist, ist nun vorgesehen, daß der Scheibenkörper 8 auf seiner auflageseitigen Oberseite 20 eine UV-durchlässige Beschichtung 21 aufweist. Dies ist in Fig. 3 dargestellt, wenngleich die Fig. 3 keine maßstabsgerechte Darstellung zeigt, da die Dicke des Scheibenkörpers 8 vorliegend etwa 8 mm beträgt, während die Dicke der Beschichtung nur zwischen 4 und 8 µm liegt. µm übrigen ist, wie die Fig. 3 verdeutlicht, lediglich auf der Oberseite 20, nicht aber auf der Unterseite 22 eine Beschichtung 21 vorgesehen. Die Beschichtung 21 ist im übrigen vollflächig auf der Oberseite 20 vorgesehen, wobei die Schichtdicke über die Fläche zumindest im wesentlichen konstant ist. Dies gilt jedenfalls bei einer ebenen Liegescheibe 3.

Im übrigen kann die Beschichtung 21 auch so ausgebildet sein, daß in verschiedenen Flächenbereichen eine größere Schichtdicke gegeben ist. Die Schichtdicke kann hier um den Faktor 1,1 bis 10 größer sein als in anderen Bereichen. Bereiche mit erhöhter Schichtdicke können insbesondere an den Stellen des Scheibenkörpers 8 vorgesehen sein, an denen der Nutzer üblicherweise auf der Liegescheibe 3 aufliegt. Dies kann beispielsweise im Kopfbereich 10, im Po- und/oder im Fußbereich vorgesehen sein.

Die Beschichtung selbst ist in jedem Falle derart ausgebildet, daß die beschichtete Liegescheibe 3 bei einer Wellenlänge von über 295 nm einen Transmissionsgrad von wenigstens 70% aufweist und diesen Transmissionswert auch während einer Bestrahlungsdauer von über 1000 Stunden nicht unterschreitet. Dies gilt auch dann, wenn die beschichtete Liegescheibe 3 beidseitig beschichtet ist.

Die Beschichtung selbst weist ein polysiloxanbasiertes Lacksystem auf. Mit dem Lacksystem ist ein Haftvermittler kombiniert. Der Haftvermittler wird vor dem Aufbringen des Lacksystems auf die Oberseite 20 des Scheibenkörpers 8 mit dem Lacksystem vermischt. Bei dem Haftvermittler handelt es sich vorliegend um Essigsäure, die in Verbindung mit dem Lacksystem beim Beschichten dazu führt, daß sich eine minimale Aufrauhung im nm- bis µm-Bereich auf der Oberseite 20 ergibt, was wiederum dazu führt, daß die aufgebrachte Beschichtung sicher und dauerhaft am Scheibenkörper 8 anhaftet. Im übrigen führt die gleichzeitige Aufbringung des Haftvermittlers mit dem Lacksystem dazu, daß sich durch den vorgenannten Haftvermittler keine Eintrübung der Oberseite 20 der Liegescheibe 8 ergibt, was den Transmissionsgrad durch den Scheibenkörper 8 hindurch beeinträchtigen würde.

Es versteht sich, daß statt der vorgenannten Essigsäure als Haftvermittler auch andere Stoffe in Frage kommen, die einen ähnlichen Anrauhungseffekt erzeugen. Grundsätzlich können hier auch andere Säuren, Aldehyde, Ketone, Alkohole, Ester, Äther, Lösungsmittel sowie deren Mischungen und Kombinationen zum Einsatz kommen, wobei die Art des Haftvermittlers und die Menge jeweils dann so zu wählen ist, daß sich zum einen die vorgenannte Anrauhung im nm- bzw. µm-Bereich ergibt und zum anderen eine Transmissionsbeeinträchtigung des UV-Lichts nicht oder allenfalls geringfügig auftritt.

In Fig. 4 ist schematisch das erfindungsgemäße Herstellungs- bzw. Beschichtungsverfahren dargestellt. Das Verfahren beginnt vorliegend damit, daß die angelieferten Scheibenkörper 8 zunächst in eine nicht dargestellte Transporteinrichtung eingehängt werden. Dies ist im Schritt A dargestellt. Zum Einhängen können die in der Liegescheibe 3 vorgesehenen Öffnungen 5 verwendet werden. Hinzuweisen ist darauf, daß die Beschichtung des Scheibenkörpers 8 im verformten Zustand des Scheibenkörpers 8 erfolgt, sofern der Scheibenkörper 8 entsprechende Verformungen aufweist. Letztlich handelt es sich bei den beim erfindungsgemäßen Verfahren zu beschichtenden Scheibenkörpern um schon fertige Liegescheiben, die beim Stand der Technik eingesetzt werden.

Nach dem Einhängen in die Transporteinrichtung wird die gesamte Liegescheibe 8 mit Ethanol vorgereinigt. Dies kann per Hand mit entsprechenden Tüchern oder maschinell erfolgen, wobei darauf zu achten ist, daß die Scheiben bei der mechanisch-chemischen Reinigung nicht zerkratzt werden. Dies erfolgt im Verfahrensschritt B.

Anschließend erfolgt die Entfernung von Staub und anderen noch an der Oberfläche anhaftenden Partikeln sowie die statische Entladung des Scheibenkörpers. Hierzu wird ionisierte Druckluft mit einem Druck von ca. 8 bar auf die Oberseite 8 aufgeblasen. Dies erfolgt im Schritt C.

Im Schritt D erfolgt die Beschichtung mit einem Polysiloxan-Lacksystem im Flutverfahren. Das Polysiloxan-Lacksystem ist dabei mit Essigsäure als Haftvermittler kombiniert, so daß ein gemeinsamer Auftrag erfolgt. Das Beschichtungsmaterial wird an der obersten Stelle des Scheibenkörpers 8 aufgegeben und läuft dann an der Oberseite 20 entlang. Das reine Befluten einer Seite dauert üblicherweise zwischen 5 bis 10 Minute.

Ist lediglich eine einseitige Beschichtung des Scheibenkörpers 8 vorgesehen, kann anschließend zum nachfolgend beschriebenen Schritt E übergegangen werden. Ist eine beidseitige Beschichtung des Scheibenkörpers 8 vorgesehen, wird der Beschichtungsvorgang auf der noch unbeschichteten Seite im Flutverfahren wiederholt.

Im übrigen kann die Beschichtung im Schritt D, insbesondere wenn eine beidseitige Beschichtung des Scheibenkörpers 8 vorgenommen werden soll, die Beschichtung auch im Tauchverfahren erfolgen. Statt des Tauchverfahrens kann grundsätzlich auch noch ein anderes Beschichtungsverfahren eingesetzt werden, beispielsweise das Sprühverfahren, bei dem Scheibenkörper 8 ein- oder beidseitig durch Besprühen beschichtet wird.

Nach Beendigung der Beschichtung erfolgt im Schritt E ein Ablüften des beschichteten Scheibenkörpers 8, und zwar so lange, bis das Lacksystem bzw. die Beschichtung 21 staubtrocken ist. Das Ablüften erfolgt bei Raumtemperatur.

Nach dem Ablüften wird die beschichtete Liegescheibe 3 im Schritt F thermisch ausgehärtet. Dies erfolgt in einem Trocknungsraum. Das thermische Aushärten erfolgt bei einer Temperatur von ca. 90°C.

Nach dem Aushärten und Abkühlen der Liegescheibe wird diese im Schritt G von der Transporteinrichtung genommen, verpackt und für den Versand vorbereitet.

### Bezugszeichenliste:

- 1: Bräunungsgerät
- 2: Unterbau
- 3: Liegescheibe
- 4: Oberteil
- 5: Bräunungsleuchte
- 6: Gresichtsbräuner
- 7: Schutzscheibe
- 8: Scheibenkörper
- 9: Vorsprung
- 10: Vorsprung
- 11: Öffnung
- 12: Kopfbereich
- 13: Vertiefung
- 14: Zentrum
- 15: Abstand
- 16: Stirnkante
- 17: Erhöhung
- 18: Bereich
- 19: Abschrägung
- 20: Oberseite
- 21: Beschichtung
- 22: Unterseite

- A bis G: Verfahrensschritte
- M: Mittelachse

## Patentansprüche

1. Liegescheibe (3) für ein Bräunungsgerät (1), mit einem UV-durchlässigen Scheibenkörper (8) aus Kunststoff zur Auflage eines Nutzers,
**dadurch gekennzeichnet,**
**daß** der Scheibenkörper (8) auf seiner Oberseite (20) und seiner Unterseite (22) eine UV-durchlässige Beschichtung (21) aufweist, und daß die Beschichtung (21) bei einer Strahlung im Wellenlängenbereich zwischen 280 nm und 450 nm einen Transmissionsgrad zwischen 60 und 90% aufweist.

2. Liegescheibe nach Anspruch 1, daß die Beschichtung (21) bei einer Wellenlänge von 295 nm einen Transmissionsgrad von mehr als 70% aufweist.

3. Liegescheibe nach Anspruch 2, **dadurch gekennzeichnet, daß** die Beschichtung (21) derart ausgebildet ist, daß der Transmissionsgrad über eine Bestrahlungsdauer von wenigstens 100 Stunden, insbesondere bis 1000 Stunden nicht unterschritten wird.

4. Liegescheibe nach einem der vorhergehenden Ansprüche, **dadurch gekennzeichnet, daß** die Beschichtung (21) ein kunststoffbasiertes Lacksystem aufweist, insbesondere auf Basis zumindest eines organischen und/oder anorganischen Polymers ausgebildet ist und daß, vorzugsweise das Lacksystem Silikon- oder Polysiloxan enthält oder hieraus besteht oder silikon- oder polysiloxanbasiert ist.

5. Liegescheibe nach einem der vorhergehenden Ansprüche, **dadurch gekennzeichnet, daß** der Scheibenkörper (8) aus einem thermoplastischen Kunststoff, insbesondere PMMA, besteht oder diesen umfaßt und daß, vorzugsweise, die Dicke des Scheibenkörpers (8) wenigstens 5 mm beträgt, insbesondere zwischen 6 und 12 mm liegt und bevorzugt etwa 8 mm beträgt.

6. Liegescheibe nach Anspruch 4 oder 5, **dadurch gekennzeichnet, daß** das Lacksystem mit einem Haftvermittler kombiniert ist.

7. Liegescheibe nach einem der vorhergehenden Ansprüche, **dadurch gekennzeichnet, daß** der Haftvermittler ausgewählt ist aus der Gruppe von Säuren, insbesondere organischen Carbonsäuren und vorzugsweise Essigsäure, Aldehyden, Ketonen, Alkoholen, Estern, Äthern, Lösungsmitteln, sowie deren Mischungen und Kombinationen.

8. Liegescheibe nach einem der vorhergehenden Ansprüche, **dadurch gekennzeichnet, daß** die Schichtdicke der Beschichtung (21) kleiner 50 µm ist, vorzugsweise zwischen 2 und 10 µm beträgt.

9. Bräunungsgerät (1) mit einer Liegescheibe (3) nach einem der vorhergehenden Ansprüche.

10. Verfahren zur Herstellung einer Liegescheibe (3) für ein Bräunungsgerät (1), wobei die Liegescheibe (3) einen UV-durchlässigen Scheibenkörper (8) aus Kunststoff aufweist und wobei der Scheibenkörper (8) auf seiner Oberseite (20) und seiner Unterseite (22) mit einer UV-durchlässigen Beschichtung (21) beschichtet wird.

11. Verfahren nach Anspruch 10, **dadurch gekennzeichnet, daß** der Scheibenkörper (8) auf seiner Oberseite (20) im Flutverfahren oder Sprühverfahren beschichtet wird.

12. Verfahren nach Anspruch 10 und 11, **dadurch gekennzeichnet, daß** die Oberseite (20) des Scheibenkörpers (8) vor dem Beschichten insbesondere mit einem Alkohol und/oder mit ionisierter Druckluft gereinigt und/oder statisch entladen wird.

13. Verfahren nach einem der vorhergehenden Verfahrensansprüche, **dadurch gekennzeichnet, daß** die Beschichtung (21) nach dem Aufbringen bei einer Temperatur unter 40°C, insbesondere bei Raumtemperatur, ablüftet und getrocknet wird.

14. Verfahren nach einem der vorhergehenden Verfahrensansprüche, **dadurch gekennzeichnet, daß** der Scheibenkörper (8) mit aufgebrachter Beschichtung (21) bei einer Temperatur größer 60°, insbesondere zwischen 80°C und 100°C, ausgehärtet wird.

## Claims

1. Lying pane (3) for a tanning device (1), comprising a UV-permeable pane body (8) made from plastic for supporting a user, **characterised in that** the pane body (8) has on its upper side (20) and on its underside (22) a UV-permeable coating (21), and **in that** the coating (21) has a transmittance of between 60 and 90% for radiation in the wavelength range between 280 nm and 450 nm.

2. Lying pane according to claim 1, **characterised in that** the coating (21) has a transmittance of more than 70% for a wavelength of 295 nm.

3. Lying pane according to claim 2, **characterised in that** the coating (21) is configured in such a way that there is no drop below the transmittance for an irradiation period of at least 100 hours, in particular up to 1000 hours.

4. Lying pane according to one of the preceding claims, **characterised in that** the coating (21) comprises a plastic-based lacquer system, in particular is formed on the basis of at least one organic and/or inorganic polymer, and **in that** preferably the lacquer system contains or consists of silicone or polysiloxane or is based on silicone or polysiloxane.

5. Lying pane according to one of the preceding claims, **characterised in that** the pane body (8) consists of or comprises a thermoplastic, in particular PMMA, and **in that** preferably the thickness of the pane body (8) is at least 5 mm, in particular is between 6 and 12 mm and preferably is around 8 mm.

6. Lying pane according to claim 4 or 5, **characterised in that** the lacquer system is combined with an adhesion promoter.

7. Lying pane according to one of the preceding claims, **characterised in that** the adhesion promoter is selected from the group consisting of acids, in particular organic carboxylic acids and preferably acetic acid, aldehydes, ketones, alcohols, esters, ethers, solvents, and mixtures and combinations thereof.

8. Lying pane according to one of the preceding claims, **characterised in that** the layer thickness of the coating (21) is less than 50 µm, preferably between 2 and 10 µm.

9. Tanning device (1) comprising a lying pane (3) according to one of the preceding claims.

10. Method for producing a lying pane (3) for a tanning device (1), wherein the lying pane (3) comprises a UV-permeable pane body (8) made from plastic, and wherein the pane body (8) is coated on its upper side (20) and on its underside (22) with a UV-permeable coating (21).

11. Method according to claim 10, **characterised in that** the pane body (8) is coated on its upper side (20) by means of a flow coating process or spray coating process.

12. Method according to claims 10 and 11, **characterised in that** the upper side (20) of the pane body (8) is statically discharged and/or cleaned in particular with an alcohol and/or with ionised compressed air prior to coating.

13. Method according to one of the preceding method claims, **characterised in that** the coating (21) after being applied is flashed off and dried at a temperature below 40°C, in particular at room temperature.

14. Method according to one of the preceding method claims, **characterised in that** the pane body (8) with the applied coating (21) is cured at a temperature above 60°C, in particular between 80°C and 100°C.

## Revendications

1. Banquette de couchage (3) pour un banc solaire (1) avec un corps de vitre (8) en plastique transparent aux UV permettant à un utilisateur de s'allonger,
**caractérisée en ce que**
le corps de vitre (8) comprend sur sa face supérieure (20) et sur sa face inférieure (22) un revêtement (21) transparent aux UV et **en ce que** le revêtement (21) présente pour un rayonnement dans l'intervalle de longueur d'onde entre 280 nm et 450 nm un degré de transmission compris entre 60 et 90 %.

2. Banquette de couchage selon la revendication 1, **caractérisée en ce que** le revêtement (21) présente pour une longueur d'onde de 295 nm un degré de transmission de plus de 70 %.

3. Banquette de couchage selon la revendication 2, **caractérisée en ce que** le revêtement (21) est configuré de telle sorte que le degré de transmission n'est pas dépassé par une valeur inférieure pendant une durée d'exposition au rayonnement d'au moins 100 heures, en particulier de jusqu'à 1000 heures.

4. Banquette de couchage selon l'une quelconque des revendications précédentes, **caractérisée en ce que** le revêtement (21) comporte un système de laque à base de plastique, en particulier est constitué à base d'au moins un polymère organique et/ou inorganique, et **en ce que**, de préférence, le système de laque contient des silicones ou des polysiloxanes ou en est constitué, ou **en ce qu'**il est à base de silicones ou de polysiloxanes.

5. Banquette de couchage selon l'une quelconque des revendications précédentes, **caractérisée en ce que** le corps de vitre (8) consiste en un produit synthétique thermoplastique, en particulier en PMMA, ou en comporte, et **en ce que**, de préférence, l'épaisseur du corps de vitre (8) est d'au moins 5 mm, en particulier est comprise entre 6 et 12 mm, et est de préférence d'environ 8 mm.

6. Banquette de couchage selon la revendication 4 ou 5, **caractérisée en ce que** le système de laque est combiné à un agent de pontage.

7. Banquette de couchage selon l'une quelconque des revendications précédentes, **caractérisée en ce que** l'agent de pontage est choisi parmi le groupe des acides, en particulier des acides organiques carboxyliques et de préférence l'acide acétique, des aldéhydes, des cétones, des alcools, des esters, des éthers, des solvants, de même que leurs mélanges et leurs combinaisons.

8. Banquette de couchage selon l'une quelconque des revendications précédentes, **caractérisée en ce que** l'épaisseur de couche du revêtement (21) est inférieure à 50 µm, qu'elle est de préférence comprise entre 2 et 10 µm.

9. Banc solaire (1) avec une banquette de couchage (3) selon l'une quelconque des revendications précédentes.

10. Procédé pour la fabrication d'une banquette de couchage (3) pour un banc solaire (1), dans lequel la vitre de couchage (3) comporte un corps de vitre (8) transparent aux UV, en plastique, et dans lequel le corps de vitre (8) est revêtu à sa face supérieure (20) et à sa face inférieure (22) d'un revêtement (21) transparent aux UV.

11. Procédé selon la revendication 10, **caractérisé en ce que** le corps de vitre (8) est revêtu à sa face supérieure (20) au cours d'un procédé par immersion ou d'un procédé par pulvérisation.

12. Procédé selon les revendications 10 et 11, **caractérisé en ce qu'**avant le revêtement, la face supérieure (20) du corps de vitre (8) est nettoyée et/ou déchargée statiquement, en particulier avec un alcool et/ou avec de l'air comprimé ionisé.

13. Procédé selon l'une quelconque des revendications précédentes portant sur le procédé, **caractérisé en ce qu'**après l'application, le revêtement (21) est ventilé et séché à une température en dessous de 40 °C, en particulier à la température ambiante.

14. Procédé selon l'une quelconque des revendications précédentes portant sur le procédé, **caractérisé en ce que** le corps de vitre (8), avec le revêtement (21) appliqué dessus, est durci à une température supérieure à 60°, en particulier entre 80 °C et 100°C.
